# EUROPEAN PATENT SPECIFICATION

(11) **EP 2 847 582 B1**
(45) Date of publication and mention of the grant of the patent: **04.04.2018**
(21) Application number: 13727971.7
(22) Date of filing: 10.05.2013
(51) Int. Cl.: G01N 27/72, G01N 33/24, C22B 1/00

(54) **ORE ANALYSIS SYSTEM**
ERZANALYSESYSTEM
SYSTÈME D'ANALYSE DE MINERAIS

(30) Priority: 10.05.2012 ZA 201203399
(43) Date of publication of application: 18.03.2015
(73) Proprietor: Sandvik Mining And Construction RSA (Pty) Ltd, 1462 East Rand (ZA)
(72) Inventor: LEPPANEN, Jarmo, 1462 East Rand (ZA); OOSTHUIZEN, Ockert, 1462 East Rand (ZA)
(74) Representative: Lyytinen, Veera Elise
(86) International application number: PCT/IB2013/000901
(87) International publication number: WO 2013/167960

(56) References cited:
- EP-A1- 0 043 826
- JP-A- S57 144 455
- US-A- 2 760 769
- US-A- 3 433 057

## Description

### FIELD OF THE INVENTION

The invention relates to detecting one or more target elements contained within particulate material. More specifically, the invention relates to analysing ore and detecting designated content in it.

### BACKGROUND OF THE INVENTION

During geological surveys, prospecting and similar activities a capability to analyse rock cuttings and the like on a continuous basis would be of significant value. For example, drilling could be directly controlled in response to information produced by an analyser. The tedious process of collecting cores or samples which are subsequently analysed in a borehole would be avoided.

Any system for analysing ore and for detecting designated content relies on evaluating or detecting some physical aspect or chemical property, which is a function of ore quality. Diverse techniques which have been used for this purpose include systems which are responsive to identified characteristics or factors which display, under certain conditions, defined responses. These attributes or characteristics include, at least, the following: a defined chemical reaction, spectral analysis, magnetic properties, photometric properties, x-ray analysis, magneto-optical analysis, conductivity properties, gamma radiation and density and hardness factors or values.
Each approach has benefits and drawbacks. For example, a photometric sorter is responsive to surface characteristics and cannot detect the presence of elements which are not expressed on a surface of a particle. Similar limitations can exist with x-ray and magneto-optical techniques. Spectral analysis is accurate but normally is carried out under laboratory conditions. A general commentary on the various techniques can be found in the prior art and for this reason is not repeated here. Broadly it can be stated that some approaches are time consuming, require the use of specialised equipment and are best implemented under laboratory conditions. Other approaches are element-specific. For example, a technique designed to detect iron cannot easily be adapted to detect the presence, say, of copper.

It is known from EP0043826B1 to use two sensing coils to analyse magnetic properties of ore samples falling through the magnetic field of a primary coil.

### SUMMARY OF THE INVENTION

An object of the present invention is to provide an ore analyser which can be used to detect the presence of a number of different target elements rapidly and effectively, which can be made in a robust, easily transportable form and which lends itself to use directly in conjunction with rock drilling and exploration equipment.
A material can be categorised as a ferromagnetic, paramagnetic or diamagnetic material, depending on the susceptibility of the material to a magnetic field. An analyser which is intended to detect ferromagnetic and paramagnetic elements is, however, subject to certain constraints, some of which can be addressed by suitable electronic designs. The analyser should also be kept away from magnetic materials e.g. steel and iron.

In a mining application, where the analyser could be called upon to detect the presence of a number of different target elements rapidly and effectively, it is essential that the analyser should be compact, robust and easily transportable. These attributes must also be possessed by equipment which handles rock cuttings produced by a mining machine and which allows the rock cuttings to be directed to the analyser. Preferably, such equipment should be integrated with the analyser to produce a compound device which can be adapted for use with a variety of different mining machines and which is capable of being used, without undue precautions being taken to protect the device, in mining environments.

When an atom is subjected to a magnetic field, the statistical distribution of the electrons can be modified in such a way that the atom starts exhibiting a magnetic field of its own. This field is usually only present while the applied field is present, with the exception of ferromagnetic materials where the field can remain afterwards. By detecting the magnetic fields as described, the presence of specific elements can be detected. These elements are those that exhibit ferromagnetism or strong paramagnetism. Elements with this property occur when specific electron sub-shells are unfilled on the specific atom. The important electron level for the specific technique being used by the invention is the valence level, that is, the last or highest energy level in which the specific element has electrons. This level will be unfilled for all but the noble gases, and for metals it will have less than half of the possible allowed electrons. Due to the unfilled state of this level, it will be easier to change the spatial orientation of its electrons by applying a magnetic field to the atom. These elements can be detected by this invention.
The invention is concerned inter alia with an analyser which allows a desired characteristic to be detected whereby at least ferromagnetic and paramagnetic elements can be identified.
The analyser includes an exciting annular coil, a first sensing annular coil and a second sensing annular coil, wherein the exciting coil surrounds at least part of a pathway for a particulate sample and is located between the first and second sensing coils, wherein each coil is respectively wound on an axis which is aligned with, and which is centrally positioned in, the pathway, a signal generator which supplies an exciting signal to the exciting coil which thereby establishes an electromagnetic field in at least part of the pathway, a receiver which detects a first signal in the first sensing coil and a second signal in the second sensing coil which are produced by passage of the sample on the pathway, and a processor which produces an output signal which is dependent on a differential between the first and second signals and in which interference signals are substantially eliminated.

The output signal is representative of the magnetic susceptibility of the sample.

The analyser is highly sensitive and for this reason, at least, extraneous effects which could influence the output signal should be eliminated as far as is possible. The effect of thermal drift, in particular, on the output signal can be substantial. To address this each coil is preferably wound on a former which has substantial thermal dimensional stability. A suitable former for use with each respective coil is made from borosilicate. Preferably each former is made from quartz glass.

To address the effects of thermal drift (including thermal dimensional stability of the coils and the formers) yet further, at least the coils and the formers may be enclosed in thermally-stabilized thermal sink. This may be achieved by enclosing the coils and formers in a casing which is filled with a liquid. The temperature of the liquid is accurately controlled in response to suitable sensors. Alternatively a liquid heated to a suitable temperature which should correspond to the optimum operating temperature of the coils and the electronics in the analyser, is circulated through the casing. The liquid should have a substantial mass relative to the mass of the coils and formers. If the temperature of the liquid is accurately controlled then the mass of liquid acts as a thermal sink which helps to absorb and negate temperature fluctuations in the coils and formers.

An object passing through the analyser will generate two pulses in the analyser. A first pulse arises when the object enters the electromagnetic field of the analyser, and a second pulse is generated as the object leaves the electromagnetic field. The pulses are substantially at the same level but, in general terms, have opposite phases. The pulses can be combined electronically to generate a difference signal, either in phase or in amplitude, as the case may be.

If the coils are axially aligned then the transmitting (exciting) coil is positioned between the two receiving (sensing) coils so that when the coils are vertically orientated, an object falling under gravity action will move in an axial direction in succession through the aligned coils. By suitable design the electromagnetic field produced by the transmitting coil, in the region of an upper sensing coil, will be the same as the electromagnetic field produced by the transmitting coil at a lower sensing coil. However, the object, as it traverses the lower coil, will move at a slightly greater speed than the speed at which it traverses the upper coil.

If the coils are radially aligned then the sensing coils are simultaneously responsive to the passage of the particle. Effects on the sensing coils due to speed differences are, automatically, eliminated. However, with the transmitting coil positioned between an inner sensing coil and an outer sensing coil the electromagnetic fields on the inner and outer sensing coils differ slightly.

In each embodiment signals from the sensing coils are combined and processed. The received signals are adjusted for static field imbalances, based on information of the transmitted electromagnetic field, and on a long-term received signal from the sensing coils. A short-term value which is dependent on the size and magnetic susceptibility of the particular object is generated.

It is possible, particularly if a continuous stream of sample material is being analysed, for the sample material to be passed through the analyser in a direction which is at a right angle to a longitudinal axis around which the coils are arranged. For example the material stream can pass between the exciting coil and one of the receiving or sensing coils. A disadvantage of this configuration is that the analyser must be calibrated at regular intervals to compensate for drift effects.

The exact spatial relationship of the transmitting or exciting coil and of the two receiving coils may be varied depending on the application but should always be such that the direct effect of the exciting coil (i.e. the effect of the exciting coil in the absence of any sample) is cancelled by combining the signals from the receiving coils.

As the field strength inside each coil is relatively constant from the middle to the edges of its sensing area, the signal produced by each coil is not significantly affected if a sample moving through a coil is offset from the centerline.

Although the three coils can be positioned relative to one another in various configurations, in a first practical implementation the coils are configured in an axial arrangement around a longitudinal axis. Each coil surrounds the axis and the coils are spaced from one another in an axial direction. The exciting coil is then positioned between the two receiving coils. It has however been found, through experimentation, that under certain conditions the axial arrangement of coils does not give the same level of performance as a radial coil configuration.

Thus, in a preferred embodiment, the coils are positioned in a radial configuration which lies in a horizontally-extending plane. The pathway then preferably extends vertically.

The ore analyser further includes a material handling system which is partly positioned upstream of the coils, to feed samples along the pathway through the coils. The samples may be fed substantially continuously and the samples may fall under gravity action.

Preferably the materials handling system includes:
a) an apparatus for separating a stream of rock particles produced by a mining machine into fine material, which is directed to waste, and rock cuttings which are coarser than the fine material;
b) a first guide structure, made from a non-magnetic material, which has an upper end connected to the apparatus and a lower end and which encloses rock cuttings falling, from the apparatus, under gravity action;
c) a controller at the lower end which collects the falling rock cuttings and which then causes the rock cuttings to move at a controlled speed along the pathway whereby the cuttings are presented to the coils whereafter the rock cuttings leave the coils at an exit; and
d) a second guide structure, made from a non-magnetic material, which has an upper end in register with the exit from the pathway and a lower end and which encloses rock cuttings falling, from the pathway, under gravity action.

At the lower end of the second guide structure, according to requirement, the rock cuttings can be directed to waste or they can be collected for further assaying or sampling using different techniques.

The apparatus for separating the rock particles may be of any appropriate kind and preferably includes a cyclone or a hydro cyhclone. The fine material, produced upon separation of the rock particles, typically includes dust and small rock cuttings, grit and the like.

If use is made of a cyclone or a hydro cyclone then it is important to maintain the correct air pressure within the system. An air, or dry, cyclone is normally kept under reduced air pressure. By way of contrast a hydro cyclone is slightly pressurised. In either case an air leakage would adversely affect the working of the cyclone. With a dry cyclone, to maintain the required level of reduced air pressure within the system, the first guide structure is connected in a leak-proof manner to an outlet from the cyclone through which the coarse rock cuttings are discharged. The first guide structure may be generally conical, tapering inwardly from the upper end to the lower end. The lower end of the first guide structure may be connected in a leak-proof manner to the analyser and, more particularly, to the pathway.

Similarly, the second guide structure may be connected in a leak-proof manner to the exit from the pathway.

A lower end of the second guide structure is preferably sealed in a suitable manner which allows rock cuttings to be collected and, thereafter, released, in a controlled manner. This can be done in different ways. It is preferred, however, that the rock cuttings, at the lower end, are collected by means of a device which has an outlet which is closed when material in the device has a mass less than a predetermined level and which opens, automatically, when the mass of material inside the device exceeds the predetermined level. This objective can be achieved by means of a mass-dependent valve e.g. a flexible tube which, normally, is biased to a closed position and which opens automatically when subjected to an internal force in excess of a predetermined magnitude thereby allowing material to be discharged from the tube under gravity action. If a hydro cyclone is used then an orifice on the housing of the hydro cyclone provides an exit for the cuttings. This replaces the mass dependent valve which is used with the so-called dry cyclone. The orifice is dimensioned to match the quantity of material flow and the design of the cyclone structure.

The controller may be of any appropriate kind. Material which falls under gravity action through the first guide structure exits at a speed which is dependent on the spacing between the upper end and the lower end of the first guide structure i.e. its axial length. The analyser should not be exposed to magnetic materials and for this reason the first guide structure is positioned to ensure that relevant components of the system are well displaced from the analyser. However, as the displacement distance increases, the speed at which the cuttings reach the controller also increases. The controller is therefore designed to intercept the falling rock cuttings, and then to release the rock cuttings at a controlled rate and at a much reduced speed, to move along the pathway.

The controller, in one form of the invention, includes a tubular member which, in use, is vertically aligned and a flexible conical component, mounted to the tubular member which, in the absence of cuttings on an outer surface, prevents rock cuttings from moving under gravity action along the pathway. The cuttings accumulate on the outer surface. The net mass of the rock cuttings gradually increases and when a force of a predetermined magnitude is exerted on the outer surface the conical component deflects and allows rock cuttings to move along the pathway.

As the workings of the coils can be affected by the presence of magnetic materials, no metals are used upstream or downstream of the coils over specific distances.

The ore analyser may for example include a first structure which encloses the pathway for samples approaching the coils, and a second structure which is positioned downstream of the coils, enclosing the pathway for samples which leave the coils. Each structure may for example be made from a plastics material.

It has been established that the analyser is responsive to the speed at which a particle moves along the pathway. As the particle accelerates under gravity action it is preferable therefore that the particle should not fall to such an extent that its speed becomes unacceptably high. Generally a maximum spacing (fall distance) is of the order of 500mm.

A particular benefit of the radial configuration lies in the fact that the speed of a sample, as it traverses the active region of each coil, is the same for all coils. The exciting coil establishes an electromagnetic field with which the sample interacts as the sample moves through the field. The signals which are produced by the first and second coils, as a result of the interaction, arise at the same time and are representative of the effect of the particle, at a particular speed, for each coil. This means that variations in the first and second signals which otherwise might be due to variations in the speed of a sample are eliminated. Such speed variations would arise, for example, if the sample went through the three coils in succession and not simultaneously.

The invention further extends to a rock drilling rig including an analyser described above.

The invention also provides a method of analysing a sample to detect a characteristic in the sample, the method including the steps of:
defining a pathway along which the sample is moved,
establishing an electromagnetic field in at least part of the pathway,
detecting a first variation in the electromagnetic field, at a first location, caused by passage of the sample along the pathway,
generating a first signal which is representative of the first variation,
detecting a second variation in the electromagnetic field, at a second location which is spaced from the first location, caused by passage of the sample along the pathway,
generating a second signal which is representative of the second variation,
producing an output signal which is dependent on a differential between the first and second signals, in which interference signals are substantially eliminated, and which output signal is representative of the magnetic susceptibility of the sample.
The invention also provides a computer program product directly loadable into the internal memory of a digital computer, comprising software code portions configured to cause an analyser as described above to perform the steps of:
establishing an electromagnetic field in at least part of the pathway,
detecting a first variation in the electromagnetic field, at a first location, caused by passage of the sample along the pathway,
generating a first signal which is representative of the first variation,
detecting a second variation in the electromagnetic field, at a second location which is spaced from the first location, caused by passage of the sample along the pathway,
generating a second signal which is representative of the second variation, and
producing an output signal which is dependent on a differential between the first and second signals, and which is indicative of a desired characteristic in the sample,
when said product is run on a computer.

### BRIEF DESCRIPTION OF THE DRAWINGS

The invention is further described by way of example which reference to the accompanying drawings in which:
Figure 1 is a side view, partly sectioned, illustrating an analyser according to a first form of the invention through which particulate samples, derived from a materials handling system, are passed;
Figure 2 is a block diagram representation of components included in the analyser;
Figure 3 illustrates from one side and in cross-section a material handling system which is constructed together with the ore analyser on an integrated basis;
Figure 4 shows another form of the analyser of the invention; and
Figure 5 is a flowchart representation of a method of detecting one or more target elements, contained within particulate material, in a substantially continuous manner on a real-time basis, in accordance with the invention.

### DESCRIPTION OF PREFERRED EMBODIMENTS

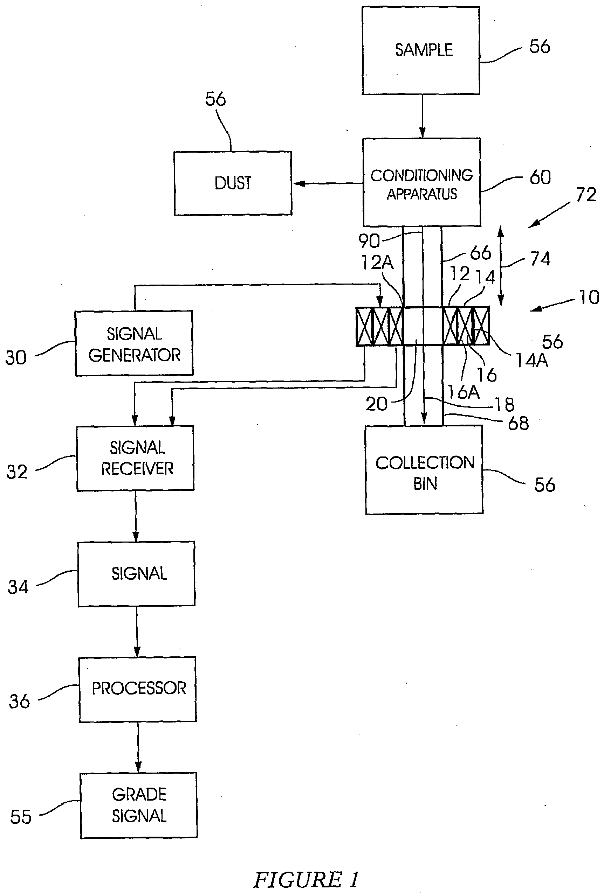

Figure 1 of the accompanying drawings illustrates from one side and in cross-section an analyser 10 according to the invention. The analyser includes first and second sensing coils 12 and 14 respectively and a third coil 16 which is referred to as a transmitting or exciting coil.

Each coil is annular and has windings which are wound on a respective ringshaped former 12A, 14A and 16A respectively. The former is chosen with care so that, to a maximum extent, it is thermally passive. Thus the former does not change dimensionally to a meaningful extent over a significant temperature range. One material which is reasonably suitable for use, in the construction of this type of former, is borosilicate. The applicant has, however, established that quartz glass is more stable and, for this reason at least, each former is made preferably from quartz glass. Certain ceramic materials are also suitable. It is observed in this connection that commercial off-the-shelf borosilicate glass and a number of commercial ceramic materials have substantially similar thermal stability characteristics. Quartz glass on the other hand is more stable but more expensive. Some ceramic materials have zero thermal expansion factors but are highly expensive.

To eliminate thermal drift effects yet further, it is preferred that the analyser should be kept in an operational state for extended periods even if it is not being used. Thus, if there is a natural tendency for the analyser to increase in temperature while it is being used, it is preferable to keep the analyser at an elevated temperature so that any thermal drift effect can be substantially eliminated by calibrating readings which are produced by the sensing coils. This approach is adopted even if the formers for the coils are made from borosilicate or quartz glass.

The innermost coil 12 encloses a space 20.

The exciting coil 16 is connected to a signal generator 30. The windings of the sensing coils 12 and 14 are connected to a receiver 32 which outputs a signal 34 to a processor 36.

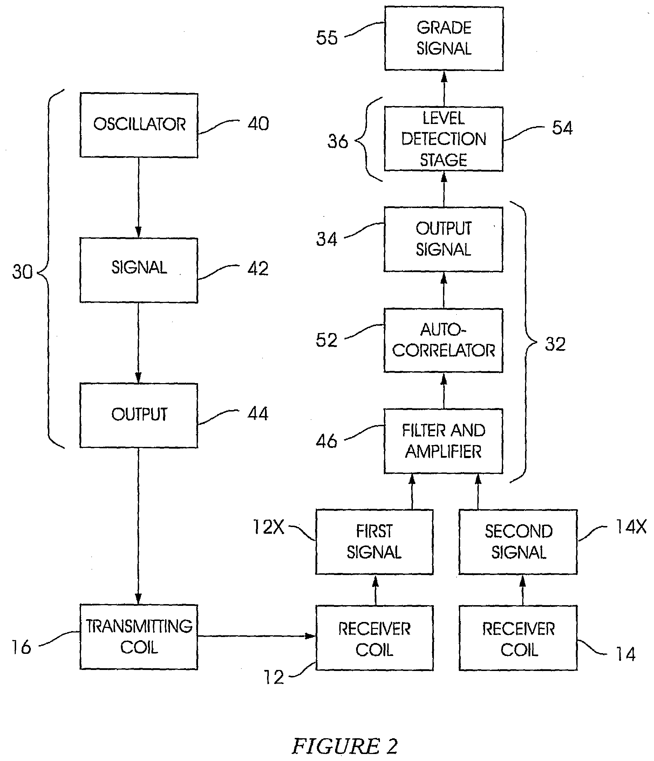

The signal generator 30 includes a quartz crystal oscillator and counter 40, a sine wave and delta modulation unit 42, and a class D output stage 44 which is connected to the exciting or transmitting coil 16 (see Figure 2).

The receiver coils 12 and 14, in response to the passage of a sample through the space 20, produce first and second signals 12X and 14X respectively, as is explained hereafter. These signals are filtered and amplified by a component 46 which is included in the receiver 32. The signals may first be digitized by being passed through an analogue to digital converter which is included in the component 46. Alternatively, digitization is carried out after the signals have been filtered.

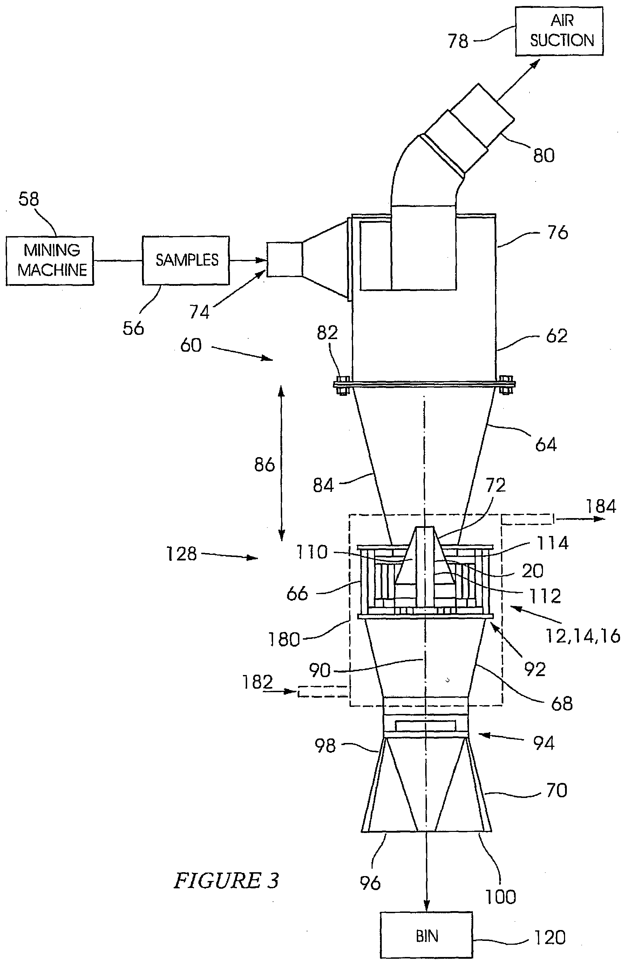

The output signal 34 is based on a differential between the signals 12X and 14X. The differential signal helps to eliminate noise and other interference which could affect the signals 12X and 14X. To achieve this differential, the signals 12X and 14X are processed by an autocorrelator 52 which produces the differential output signal 34 which is applied to a level detection stage 54 (embodied in the processor 36) which can amplify the output signal or detect when it passes a threshold or the like. The processor 36, in accordance with an algorithm and data stored in its memory, outputs a signal 55 which is representative of the characteristics of a particular ore sample e.g. its grade, material content, etc. The signal 55 is one of a plurality of signals, produced continuously by the analyser, which indicate in real time the presence or absence of a desired characteristic or characteristics in the samples. The signals provide data which can be used to control, automatically or manually, the process used to produce the samples which are presented to the analyser i.e. in a mining or exploratory process. This aspect is further described with reference to Figure 3.

Rock cuttings 56 (i.e. samples) which are produced by a rock drilling or mining machine 58 are processed in a materials handling system 60 which removes dust 62 and undersize and oversize samples - see Figure 3.

The system 60 includes apparatus 62 which is based on cyclonic principles, a first guide structure 64, an enclosure 66, a second guide structure 68, an outlet valve 70 and a controller 72.

The apparatus 62 has an inlet 74 through which rock cuttings are directed into a volute 76 of the apparatus. The cuttings are produced by the mining machine 58, which is shown schematically only. Typically the mining machine is a rock drilling machine but this is exemplary and non-limiting. An air suction source 78, notionally shown, is connected to an outlet 80 from the volute. The volute has a discharge end 82. The first guide structure 64 is connected in a leak-proof manner to the discharge end. This guide structure is in the form of a conical body 84 with an axial length 86. The body tapers inwardly in a direction away from the apparatus 62. At a lower end the body 84 is connected to the enclosure 66.

The coils 12, 14 and 16 are housed inside the enclosure 66. In order for the analyser to operate effectively rock cuttings which pass along a defined path 90, which extends through the space 20 of the analyser, should move at a relatively slow speed. Additionally, the analyser should be displaced at least by the distance 86 from magnetic materials. For this reason the enclosure 66 is made from a non-magnetic material and the first guide structure 64 and the second guide structure 68 are also made from non-magnetic materials.

The second guide structure 68 has an upper end 92 which is connected to a lower end of the enclosure 66, and a lower end 94. The outlet valve 70 is connected to the lower end. The outlet valve is made from a tubular rubber element 96. An upper end 98, of circular form, is directly connected to the end 94 of the second guide structure. A lower end 100 of the tubular element is flattened to provide a seal which is reasonably airtight. However, the lower end can open when the weight of material accumulated inside the tubular element reaches a predetermined level.

The controller 72 includes a conical component 110 which is mounted to a centrally positioned, axially aligned tube 112. A plate 114 surrounds the component 110, at an upper side of the coils. The tube 112 is concentrically positioned with respect to the pathway 90.

During operation, the suction source 78 reduces the air pressure prevailing inside the system 60. Cuttings 56 produced by the mining machine 58 are vacuumed into the volute 76 directly from the borehole which is being drilled. Dust and fine materials 62, in the entrained cuttings, coming from the mining machine, are extracted via the outlet 80. The remaining cuttings, which are relatively coarse, are forced radially outwardly onto an inner wall of the volute 76 and then slide downwardly under gravity action. The first guide structure 64 directs these coarse cuttings onto an outer surface of the conical component 110. The cuttings accumulate on the outer surface, abutting the plate 114.

The tube 112 allows the vacuum inside the materials handling system, produced by the suction source, to prevail substantially throughout the system. The likelihood that cuttings can fall directly from the volute 76 through the tube 112 is negligible for, as noted, the cuttings are forced onto the inner surface of the volute and then slide downwardly, under gravity action, on an inner surface of the conical body 84.

The mass of cuttings on the outer surface of the conical component increases as the cuttings accumulate. Ultimately a point is reached at which the conical component, which is preferably made from a relatively soft and flexible rubber, flexes inwardly and some cuttings fall through thereby moving as a continuous stream of samples 56 (Figure 1) along the pathway 90 which extends through the analyser.

The samples 56, leaving the conical component 110, then fall under gravity through the coil assembly, passing along the pathway 90.

The second guide structure 68, downstream of the coils, encloses a lower portion of the pathway 90 which extends to a collecting bin 120 for samples which have been processed.

The pathway 90 between the upper end of the structure 68 and the lower end of the structure 64 may be regarded as defining an operative area 128 of the analyser. In the absence of any sample in the operative area the excitation produced by the coil 16 produces responsive signals in the coils 12 and 14. These signals are adjusted by means of the processor 36 so that the signals are effectively cancelled i.e. the output signal 50 is, for practical purposes, zero. This approach helps to nullify the effects of unwanted interference signals, noise and the like.

The sensing field (the output of the transmitting coil 16) is generated by applying an alternating electrical signal to the coil. This signal can be a sinewave of constant frequency or can be of other form, depending on the application. The electromagnetic field can be static or time varying, depending on the specific application. Ideally the exciting coil 16 is driven with a modulated square wave signal 42 and the frequency of the signal is accurately controlled by the oscillator 40. This results in improved stability and accuracy of the sensing system.

The samples 56 fall under gravity action through the radially configured coils 12, 14 and 16. The drop distance, i.e. the axial length 86 of the structure 64, determines the speed at which the samples pass through the coils. If the speed is too high the sensitivity of the analyser is reduced - this aspect is further elaborated on hereinafter. Depending on the types of elements which are being targeted and the sizes of the cuttings or samples it might be necessary to use some mechanism which reduces the speed at which the samples pass through the coils. This allows the coils to exhibit greater sensitivity to the target elements.

The cutting samples can be presented individually (one by one) in succession to the coils. This allows a particle-by-particle determination to be made of the presence or absence of a target element. However, if the samples are passed in a continuous flow stream, through the coils, it is possible to obtain a "bulk" reading of target element content in a plurality of samples.

The coils are designed, taking into account the different radial dimensions thereof, so that, in the absence of any sample and due only to the effect of the exciting field, the coils output substantially identical signals. During a calibration phase one signal is subtracted from the other signal so that the effect of noise is eliminated. The signal 34 is then effectively zero.

The effect of an ore sample pulsing through the analyser is manifested in two ways namely, by disturbing the electromagnetic field as the sample enters a field and interacts therewith and, subsequently, by allowing the disturbance in the electromagnetic field to settle to zero as the sample leaves the operative space of the analyser.

The signals from the two sensing coils are combined and then processed by the microprocessor system which adjusts the received signals for static field imbalances based on the transmitted field information and on the long term received signal from the sensing coils, and which then produces a short term value (the signal 34) which corresponds with, or which is dependent on, the size and magnetic susceptibly of the sample.

An optical or other sensor can be used together with the coils to obtain information on the size and shape of each sample. This allows a signal to be generated which is proportional to grade, i.e. the strength of the signal is divided by a factor which is representative of the size or mass of the sample.

Due to the highly sensitive nature of the analyser of the invention unwanted thermal drift effects can have a serious negative impact on the integrity or reliability of readings produced by the analyser. In order to address the thermal drift factor the formers which hold the coils should be thermally dimensionally stable. This aspect has been referred to hereinbefore. However additional measures can be taken to counter the effects of thermal drift. One technique is to fabricate each former from a thermally stable material which is provided in skeletal form e.g. as a framework of minimal mass (material content) which nonetheless has sufficient structural rigidity to support the windings of the coils. In a general sense therefore each former could be perforated or be formed with a plurality of apertures so as to reduce its material content and thereby make the former less liable to thermally induced distortion.

A particular technique, which is intended to fall within the scope of the invention, is to enclose at least the coils and the formers which support the coils inside a casing 180 which is indicated in a dotted line in Figure 3. The casing is engaged in a leak-proof manner with an outer surface of the apparatus of the analyser against which the casing abuts. A liquid e.g. water is held inside the casing at a stable temperature. The liquid can be heated electrically, in response to thermal sensors, to a precisely controlled temperature. Alternatively, as is indicated in Figure 3, liquid from a heated source, not shown, can be directed into the casing via an inlet 182 and withdrawn from the casing via an outlet 184. Externally of the casing the temperature of the water is precisely controlled. In effect by enclosing the coils and the formers and any electronic equipment which is susceptible to thermal variations in a casing of the kind described a large thermal sink is established which can absorb and stabilise relatively minor temperature variations which might otherwise be produced by thermally sensitive components such as the formers.

The processor 36 may incorporate an algorithm for removing slowly varying content from the signal 34 which may be caused by unwanted external events.

One particular objective of the invention is to provide a facility which is capable of providing data, on a real time basis, which is indicative of the presence or absence of a target mineral, during a drilling or mining process. The data can be used either manually or automatically, e.g. through the use of a suitable processor, to aid in a mining or drilling process. Significant cost and productivity benefits are associated with this technique.

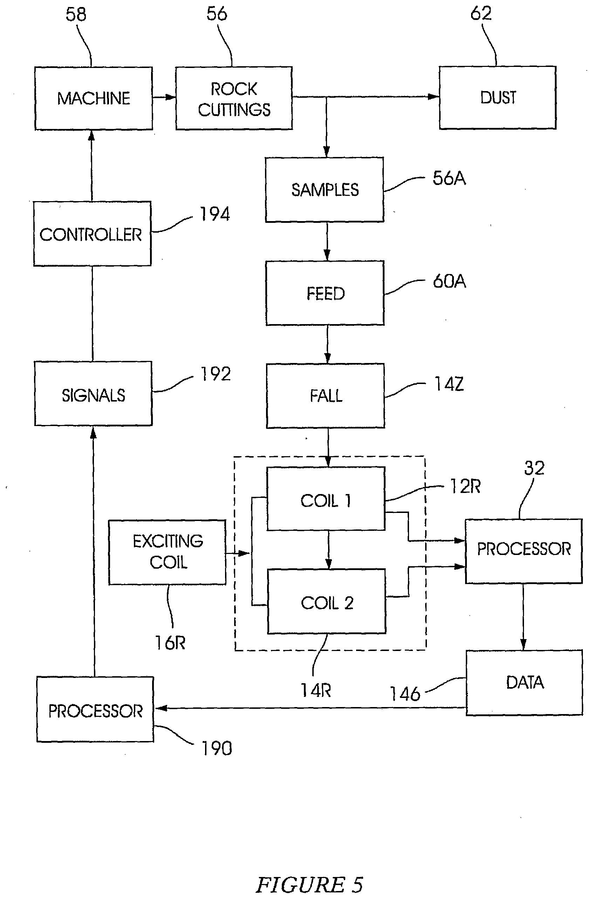

In one form of the invention the production of the grade signal 55 or any equivalent signal which reflects the presence or absence of a desired characteristic or characteristics in the rock samples under analysis, can be considered to be a satisfactory result. However, in a preferred application of the principles of the invention the grade signal 55, or any equivalent signal which is indicative of the nature of the rock cuttings which are being analysed is used, in a real time basis, to control and guide the operation of the mining machine. In this respect, referring to Figure 5, the data 146 produced by the processor 32 is further analysed in terms of a proprietary algorithm held in a secondary processor 190. In terms of this algorithm the data 146 is compared to reference data, held in a memory associated with the processor 190. The reference data is based to a substantial extent on empirical values and a number of identified parameters and variables which can directly impact on the effectiveness of a drilling or mining program. The algorithm is able to evaluate the data 146 using the reference data as a yardstick and, in response thereto, to produce output signals 192 which can be made available to an operator of the machine 58 guide the operator in the functioning of the machine. Alternatively or additionally the signals 192 are processed in an appropriate control unit 194 which interfaces with the machine 58 in order to regulate in an efficient and automatic manner the operation of the machine.

An advantage of the design is that the field strength inside the space 20 is relatively constant from the middle to the edges of the sensing area. A reading produced by the two coils 12, 14 is thus not significantly affected by the offset of a sample from the geometrical center of the coils.

Compounds of certain metals have high magnetic susceptibilities, as a result of the patterns of the electron populations for the specific metal atom. In particular, where there is a partially filled electron sub-shell or subs-shells 3d, 4f, or 5f, components with extremely high magnetic susceptibility are formed. The following metals fall within this group:

| | | |
|---|---|---|
| scandium | cerium | holmium |
| titanium | praseodymium | erbium |
| vanadium | neodymium | thulium |
| chromium | promethium | uranium |
| manganese | samarium | neptunium |
| iron | europium | plutonium |
| cobalt | gadolinium | |
| nickel | terbium | |
| copper | dysprosium | |

Many of these metals are important for the mining industry, and can be distinguished accurately from unwanted minerals (waste rock), using this property. 95% of the earth's crust consists of oxygen, silicon, aluminium, calcium, sodium, potassium, magnesium, and hydrogen. None of these elements is detected by the analyser. Thus rock which surrounds an ore deposit, as well as ore contaminants, do not have any effect on the analyser.

The analyser is capable of detecting elements inside each particulate sample. These elements, which are generally in the form of chemical compounds are, however, metallic and fall within a specific section of the periodic table. The analyser's use is not limited to the detection of pure metals however for the analyser is capable of detecting a metallic compound which has a poor electrical conductivity which is too low to be detected by a conventional metal detector. This type of compound has low or zero latent magnetism. The relevant property detected in the analyser is magnetic susceptibility. The analyser is not affected by water nor by ionic compounds or salts in a sample. Common salts in soil and ore are often compounds of sodium, calcium, magnesium, potassium and lithium and none of these elements affects the analyser in any way.

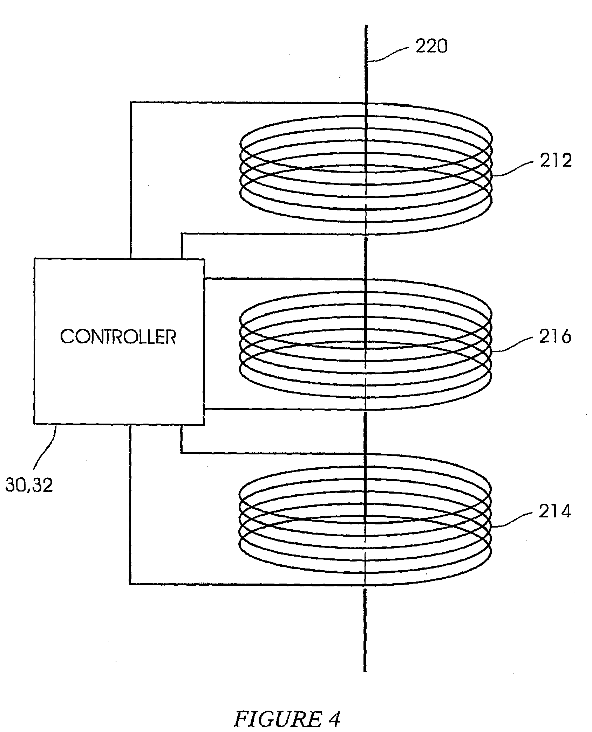

The benefits derived from the radial configuration of the coils 12, 14 and 16 (Figure 1) must be contrasted with the results achieved when similar coils 212, 214 and 216 are configured in an axially extending, vertical array, substantially as shown in Figure 4. In the axial system upper and lower coils 212 and 214 respectively are sensing coils, and a central coil 216, which is coaxially situated relative to the upper and lower coils, is an exciting coil.

In the axial configuration a material sample falls vertically under gravity action, moving generally along a longitudinal axis 220 about which the coils are positioned. An electromagnetic field produced by the exciting coil in the sensing region of the coil 212 is substantially the same as in a sensing region of the coil 214. However, the speed of a sample under test increases as it moves downwardly. The sample speed is thus higher when the sample traverses the coil 214 compared to the speed at which the sample traverses the coil 210. Despite this, in general terms the analyser shown in Figure 4 functions in the same way as the analyser with the radial coil configuration (Figure 1). The exciting signal for the electromagnetic field is generated and transmitted by the coil 216. Signals produced in the sensing coils 212 and 214 are input to the processor which amplifies the differential signal. The level and phase of the differential signal are detected, and unwanted interference signals are removed. The resulting signal is combined with other information such as sample size information in order to calculate a bulk conductivity and magnetic susceptibility value. The controller then generates an output signal (corresponding to the signal 55 in Figure 2) in a form which depends on the specific application.

The following table represents comparative results obtained when a copper sample was allowed to fall in a controlled manner from different starting points positioned at variable levels above an axial and vertical configuration of the coils (Figure 4), and above a radial configuration of the coils (as per Figure 1), respectively. With a drop height of 500mm the radial analyser gave a signal which is approximately four times the amplitude of the axial analyser. With a drop of 100mm the signal difference is of the order of a factor of two.

| Drop height | Axial analyser reading | Radial analyser reading |
|---|---|---|
| 500mm | 4.9 | 20.7 |
| 300mm | 4.9 | 21.3 |
| 100mm | 16.6 | 33.8 |

The radial configuration is thus more sensitive than the axial configuration. The effect of drift on the two types of analysers was substantially the same.

The relative higher sensitivity of the radial configuration does not necessarily mean that the radial configuration is superior in all respects to the axial configuration. For example the axial version exhibits an inherent capability of cancelling factors which could give rise to drift in output readings. If individual rock samples are to be analysed, one by one, then the axial configuration may well be highly effective. However a radial configuration is generally more suitable for sampling, on a real time basis, a continuous flow of rock cuttings, produced by a drilling machine, which are allowed to fall under gravity action through the coils. In one respect this means that when a decision is to be made on whether to use a radial coil configuration or an axial coil configuration, the application must be taken into account - for example is the analysis to be done on site under real time conditions in respect of all rock cuttings, or could the analysis be confined to a relatively slow, sample by sample, consideration?

The method of the invention can thus be implemented in at least two ways namely by using a radial coil configuration and an axial coil configuration. The flowchart in Figure 5 is applicable to each form of the method in that it shows use of a mining machine 58 to produce a plurality of rock cutting samples 56. Dust and fine materials 62 are removed from the main material stream. The resulting "cleaned" samples 56A are presented by a materials handling system 60A to the analysing equipment. Samples are allowed to fall under gravity action (block 142) and pass through an electromagnetic field 144 which is established by the exciting coil 16R. The sensing or receiving coils 12R and 14R, in an axial or radial configuration, in response to variations in the electromagnetic field caused by passage of each sample, produce respective output signals which are presented to the processor 36 which, in turn, produces data which is indicative of the presence or absence of a desired characteristic in each respective sample.

Cuttings exiting the analyser are guided by the structure 68 to the valve 70. As the mass of the cuttings collected in the valve increases a point is reached at which the flattened lower end 100 opens and cuttings are then automatically discharged from the valve. These cuttings can be directed to waste or they can be collected in the bin 120 for further assaying, sampling or the like, according to requirement.

The material handling system 60 thus is one in which particles produced by a mining machine are collected and separated into fines and coarse cuttings. The coarse cuttings are moved under gravity action continuously through the analyser 10 at a reduced speed which suits the characteristics of the analyser. The analyser is separated from magnetic components by non-magnetic guide structures. Automatic discharge of cuttings which have been exposed to the analyser is achieved in a simple manner which ensures that a degree of vacuum which is required inside the system for a cyclonic separator to operate satisfactorily, is maintained.

The system 60 can handle cuttings which are produced, say, during one drilling cycle. When a new drill rod is to be added to the drilling machine the dust collector system is stopped i.e. the vacuum source 78 is turned off. Air can then be blown through the material handling system to remove any particles which may have accumulated. The analyser is then calibrated as may be required. These steps are carried out while a new drill rod is being added to a drill string of the drilling machine 58 and the system 60 is then ready for use during a fresh drilling cycle.
The invention is also directed to a computer program product products comprising software stored on any computer useable medium. Such software, when executed in one or more data processing device or processor, causes a processor to operate as described herein. Embodiments of the invention employ any computer useable or readable medium, known now or in the future. Examples of computer useable mediums include, but are not limited to, primary storage devices (e.g., any type of random access memory), secondary storage devices (e.g., hard drives, floppy disks, CD ROMS, DVDs, ZIP disks, tapes, magnetic storage devices, optical storage devices, MEMS, nanotechnological storage device, etc.), and communication mediums (e.g., wired and wireless communications networks, local area networks, wide area networks, intranets, etc.).

## Claims

1. An analyser for detecting a desired characteristic in a rock cutting sample, the analyser including first and second sensing annular coils (12,14; 212,214), and an exciting annular coil (16;216) which surround at least part of a pathway (90) for a particulate sample (56), the exciting annular coil (16;216) being positioned between the first and second sensing coils (12,14; 212,214), a signal generator (30) which is configured to supply an exciting signal to the exciting coil (16;216) which thereby establishes an electromagnetic field in at least part of the pathway (90), a receiver (32) which is configured to detect a first signal (12X) in the first sensing coil (14;214) and a second signal (14X) in the second sensing coil (16;216) which are produced by passage of the sample on the pathway (90), **characterised in that** a processor (36) is configured to produce an output signal (34) which is dependent on a differential between the first and second signals, in which output signal interference signals are substantially eliminated, and which output signal is representative of the magnetic susceptibility of the sample.

2. The analyser according to claim 1 wherein the desired characteristic includes at least one of the following: ferromagnetism and paramagnetism.

3. The analyser according to claim 1 or 2 wherein the sample, on its passage on the pathway, causes a first pulse to be generated as the sample enters the electromagnetic field and a second pulse to be generated as the sample leaves the electromagnetic field, and wherein the processor is configured to combine the first and second pulses electronically to generate said differential.

4. The analyser according to any one of claims 1 to 3 wherein the coils are vertically orientated so that a sample falling under gravity action moves in an axial direction in succession through the aligned coils.

5. The analyser according to any one of claims 1 to 4 wherein the first and second sensing annular coils (212,214) surround at least part of the pathway (90) and are spaced in an axial sense from each other so that a sample travelling along the pathway moves first through the first sensing coil (212), then through the exciting coil (216) and then through the second sensing coil (214).

6. The analyser according to any one of claims 1 to 5 wherein the exciting coil (16) is located between the first sensing coil (12) and the second sensing coil (14) in a radial configuration.

7. A system comprising an analyser according to any one of claims 1 to 6 which further includes a material handling system (60) which is partly positioned upstream of the coils, to feed samples along the pathway (90) through the coils.

8. The system according to claim 7 wherein the materials handling system (60) includes:
a) an apparatus (62) for separating a stream of rock particles produced by a mining machine (58) into fine material, which is directed to waste, and rock cuttings which are coarser than the fine material;
b) a first guide structure (64), made from a non-magnetic material, which has an upper end connected to the apparatus and a lower end and which encloses rock cuttings falling, from the apparatus, under gravity action;
c) a controller (72) at the lower end which collects the falling rock cuttings and which then causes the rock cuttings to move at a controlled speed along the pathway (90) whereby the cuttings are presented to the coils whereafter the rock cuttings leave the coils at an exit; and
d) a second guide structure (68), made from a non-magnetic material, which has an upper end (92) in register with the exit from the pathway (90) and a lower end (94) and which encloses rock cuttings falling, from the pathway, under gravity action.

9. A method of analysing a rock cutting sample to detect a characteristic in the sample, the method including the steps of:
defining a pathway along which the sample is moved,
establishing an electromagnetic field in at least part of the pathway,
detecting a first variation in the electromagnetic field, at a first location,
caused by passage of the sample along the pathway,
generating a first signal which is representative of the first variation,
detecting a second variation in the electromagnetic field, at a second location which is spaced from the first location, caused by passage of the sample along the pathway,
generating a second signal which is representative of the second variation, **characterised in that** it comprises further producing an output signal which is dependent on a differential between the first and second signals, in which output signal interference signals are substantially eliminated, and which output signal is representative of the magnetic susceptibility of the sample.

10. The method according to claim 9 wherein the first and second locations are positioned on the pathway and are spaced apart from each other.

11. The method according to claim 9 wherein the first and second locations are located in a plane which is transverse to the pathway.

12. The method according to any one of claims 9 to 11 wherein the sample, on its passage along the pathway, generates a first pulse as the sample enters the electromagnetic field and a second pulse as the sample leaves the electromagnetic field and which includes the step of combining the first and second pulses electronically to generate said differential.

13. The method according to any one of claims 9 to 12 wherein the sample is allowed to fall under gravity action along the pathway.

14. The method according to any one of claims 9 to 13 wherein the sample is one of a plurality of samples which are directed in succession, in a continuous stream, along the pathway.

15. The method according to claim 14, which includes the steps of using a mining machine to produce the plurality of samples, removing, at least, dust from the plurality of samples, and causing the samples to move, in succession, through the electromagnetic field along a pathway, wherein a plurality of respective output signals are produced.

16. The method according to claim 15 which includes the steps of deriving measurement data from the respective output signals, comparing the measurement data to reference data to provide at least one control signal, and using the at least one control signal to control operation of a mining machine.

17. The method according to claim 16, wherein the mining machine is the mining machine used to produce the plurality of rock cutting samples.

18. A computer program product directly loadable into the internal memory of a digital computer, comprising software code portions configured to cause an analyser according to claims 1-8 to perform the steps of:
establishing an electromagnetic field in at least part of the pathway,
detecting a first variation in the electromagnetic field, at a first location, caused by passage of the sample along the pathway,
generating a first signal which is representative of the first variation,
detecting a second variation in the electromagnetic field, at a second location which is spaced from the first location, caused by passage of the sample along the pathway,
generating a second signal which is representative of the second variation, and
producing an output signal which is dependent on a differential between the first and second signals, in which output signal interference signals are substantially eliminated, and which output signal is representative of the magnetic susceptibility of the sample, when said product is run on a computer.

## Patentansprüche

1. Analysator zu dem Erfassen einer gewünschten Eigenschaft in einer Gesteinstrümmerprobe, wobei der Analysator Folgendes aufweist: erste und zweite Abtastringspulen (12, 14; 212, 214) und eine Erregerringspule (16; 216), die zumindest einen Teil eines Weges (90) für eine Partikelprobe (56) umgeben, wobei die Erregerringspule (16; 216) zwischen der ersten und der zweiten Abtastringspule (12, 14; 212, 214) positioniert ist, einen Signalerzeuger (30), der konfiguriert ist, ein Erregersignal an die Erregerspule (16; 216) zu übermitteln, die dadurch ein elektromagnetisches Feld in zumindest einem Teil des Weges (90) aufbaut, einen Empfänger (32), der konfiguriert ist, ein erstes Signal (12X) in der ersten Abtastspule (14; 214) und ein zweites Signal (14x) in der zweiten Abtastspule (16; 216) zu erfassen, die durch Verlauf der Probe über den Weg (90) produziert werden, **dadurch gekennzeichnet, dass** ein Prozessor (36) konfiguriert ist, ein Ausgabesignal (34) zu produzieren, das von einer Differenz zwischen dem ersten und dem zweiten Signal abhängig ist, wobei in dem Ausgabesignal Interferenzsignale in dem Wesentlichen eliminiert sind und wobei das Ausgabesignal für die magnetische Suszeptibilität der Probe steht.

2. Analysator nach Anspruch 1, wobei die gewünschte Eigenschaft zumindest einen der folgenden aufweist: Ferromagnetismus und Paramagnetismus.

3. Analysator nach Anspruch 1 oder 2, wobei die Probe bei ihrem Verlauf über den Weg verursacht, dass ein erster Impuls erzeugt wird, wenn die Probe in das elektromagnetische Feld eintritt, und ein zweiter Impuls erzeugt wird, wenn die Probe das elektromagnetische Feld verlässt, und wobei der Prozessor konfiguriert ist, den ersten und den zweiten Impuls elektronisch zu vereinigen, um die Differenz zu erzeugen.

4. Analysator nach einem der Ansprüche 1 bis 3, wobei die Spulen vertikal ausgerichtet sind, sodass sich eine Probe, die unter Wirkung der Schwerkraft fällt, in axialer Richtung nacheinander durch die angeordneten Spulen bewegt.

5. Analysator nach einem der Ansprüche 1 bis 4, wobei die erste und die zweite Abtastringspule (212, 214) zumindest einen Teil des Weges (90) umgeben und in einem axialen Sinne voneinander so beabstandet sind, dass sich eine Probe, die sich entlang des Weges fortbewegt, zuerst durch die erste Abtastspule (212), dann durch die Erregerspule (216) und dann durch die zweite Abtastspule (214) bewegt.

6. Analysator nach einem der Ansprüche 1 bis 5, wobei sich die Erregerspule (16) zwischen der ersten Abtastspule (12) und der zweiten Abtastspule (14) in einer radialen Konfiguration befindet.

7. System, umfassend einen Analysator nach einem der Ansprüche 1 bis 6, das weiter ein Materialhandhabungssystem (60) aufweist, das teilweise den Spulen vorgelagert positioniert ist, um Proben entlang des Weges (90) durch die Spulen einzutragen.

8. System nach Anspruch 7, wobei das Materialhandhabungssystem (60) Folgendes aufweist:
a) eine Vorrichtung (62) zu dem Trennen eines Stromes von Gesteinspartikeln, der durch eine Abbaumaschine (58) produziert wurde, in Feingut, das dem Abfall zugeführt wird, und Gesteinstrümmer, die gröber sind als das Feingut;
b) eine erste Führungsstruktur (64), hergestellt aus einem nicht magnetischen Material, das ein oberes Ende, das mit der Vorrichtung verbunden ist, und ein unteres Ende aufweist und das Gesteinstrümmer umschließt, die unter Wirkung der Schwerkraft aus der Vorrichtung fallen;
c) eine Steuerung (72) an dem unteren Ende, die die fallenden Gesteinstrümmer sammelt und die dann verursacht, dass die Gesteinstrümmer sich bei einer gesteuerten Geschwindigkeit entlang des Weges (90) bewegen, wodurch die Trümmer den Spulen vorgeführt werden, woraufhin die Gesteinstrümmer die Spulen an einem Ausgang verlassen; und
d) eine zweite Führungsstruktur (68), die aus nicht magnetischem Material hergestellt ist, die ein oberes Ende (92) in Ausrichtung mit dem Ausgang aus dem Weg (90) und ein unteres Ende (94) aufweist und die Gesteinstrümmer umschließt, die unter Wirkung der Schwerkraft aus dem Weg fallen.

9. Verfahren zu dem Analysieren einer Gesteinstrümmerprobe, um eine Eigenschaft in der Probe zu erfassen, wobei das Verfahren die folgenden Schritte aufweist:
Definieren eines Weges, den die Probe entlangbewegt wird,
Aufbauen eines elektromagnetischen Feldes in zumindest einem Teil des Weges,
Erfassen einer ersten Variation des elektromagnetischen Feldes an einer ersten Stelle, die durch Verlauf der Probe entlang des Weges verursacht wurde,
Erzeugen eines ersten Signals, das für die erste Variation steht,
Erfassen einer zweiten Variation des elektromagnetischen Feldes an einer zweiten, von der ersten Stelle beabstandeten Stelle, die durch Verlauf der Probe entlang des Weges verursacht wurde,
Erzeugen eines zweiten Signals, das für die zweite Variation steht,
**dadurch gekennzeichnet, dass** es weiter Folgendes umfasst:
Produzieren eines Ausgabesignals, das von einer Differenz zwischen dem ersten und dem zweiten Signal abhängig ist, wobei in dem Ausgabesignal Interferenzsignale in dem Wesentlichen eliminiert sind und wobei das Ausgabesignal für die magnetische Suszeptibilität der Probe steht.

10. Verfahren nach Anspruch 9, wobei die erste und die zweite Stelle auf dem Weg positioniert und voneinander beabstandet sind.

11. Verfahren nach Anspruch 9, wobei sich die erste und die zweite Stelle in einer Ebene befinden, die quer zu dem Weg verläuft.

12. Verfahren nach einem der Ansprüche 9 bis 11, wobei die Probe auf ihrem Verlauf entlang des Weges einen ersten Impuls erzeugt, wenn die Probe in das elektromagnetische Feld eintritt, und einen zweiten Impuls, wenn die Probe das elektromagnetische Feld verlässt, und das den Schritt des elektronischen Vereinigens des ersten und des zweiten Impulses aufweist, um die Differenz zu erzeugen.

13. Verfahren nach einem der Ansprüche 9 bis 12, wobei die Probe unter Wirkung der Schwerkraft entlang des Weges fallen gelassen wird.

14. Verfahren nach einem der Ansprüche 9 bis 13, wobei die Probe eine von einer Vielzahl von Proben ist, die nacheinander in einem kontinuierlichen Strom entlang des Weges gelenkt werden.

15. Verfahren nach Anspruch 14, das die folgenden Schritte aufweist: Verwenden einer Abbaumaschine, um die Vielzahl von Proben zu produzieren, Entfernen zumindest von Staub von der Vielzahl von Proben und Verursachen, dass die Proben sich nacheinander durch das elektromagnetische Feld den Weg entlangbewegen, wobei eine Vielzahl von jeweiligen Ausgabesignalen produziert wird.

16. Verfahren nach Anspruch 15, das die folgenden Schritte aufweist: Ableiten von Messdaten von den jeweiligen Ausgabesignalen, Vergleichen der Messdaten mit Referenzdaten, um zumindest ein Steuersignal bereitzustellen, und Verwenden des zumindest einen Steuersignals, um den Betrieb einer Abbaumaschine zu steuern.

17. Verfahren nach Anspruch 16, wobei die Abbaumaschine die Abbaumaschine ist, die verwendet wird, um die Vielzahl von Gesteinstrümmerproben zu produzieren.

18. Computerprogrammprodukt, das direkt auf den internen Speicher eines Digitalcomputers ladbar ist, umfassend Softwarecodeabschnitte, die konfiguriert sind, zu verursachen, dass ein Analysator nach einem der Ansprüche 1 bis 8 die folgenden Schritte durchführt:
Aufbauen eines elektromagnetischen Feldes auf zumindest einem Teil des Weges,
Erfassen einer ersten Variation des elektromagnetischen Feldes an einer ersten Stelle, die durch Verlauf der Probe entlang des Weges verursacht wurde,
Erzeugen eines ersten Signals, das für die erste Variation steht,
Erfassen einer zweiten Variation des elektromagnetischen Feldes an einer zweiten, von der ersten Stelle beabstandeten Stelle, die durch Verlauf der Probe entlang des Weges verursacht wurde,
Erzeugen eines zweiten Signals, das für die zweite Variation steht, und
Produzieren eines Ausgabesignals, das von einer Differenz zwischen dem ersten und dem zweiten Signal abhängig ist, wobei in dem Ausgabesignal Interferenzsignale in dem Wesentlichen eliminiert sind und wobei das Ausgabesignal für die magnetische Suszeptibilität der Probe steht,
wenn das Produkt auf einem Computer abgespielt wird.

## Revendications

1. Analyseur pour détecter une caractéristique désirée dans un échantillon de débris rocheux, l'analyseur incluant des première et seconde bobines annulaires de détection (12,14 ; 212,214), et une bobine annulaire d'excitation (16;216) qui entoure au moins une partie d'une voie (90) pour un échantillon particulier (56), la bobine annulaire d'excitation (16;216) étant positionnée entre les première et seconde bobines de détection (12,14 ; 212,214), un générateur de signal (30) qui est configuré pour fournir un signal d'excitation à la bobine d'excitation (16;216) qui établit ainsi un champ électromagnétique dans au moins une partie de la voie (90), un récepteur (32) qui est configuré pour détecter un premier signal (12X) dans la première bobine de détection (14;214) et un second signal (14X) dans la seconde bobine de détection (16;216) qui sont produits par passage de l'échantillon sur la voie (90),
**caractérisé en ce qu'**un processeur (36) est configuré pour produire un signal de sortie (34) qui est dépendant d'un différentiel entre les premier et second signaux, dans lequel des signaux d'interférence de signal de sortie sont sensiblement éliminés, et lequel signal de sortie est représentatif de la susceptibilité magnétique de l'échantillon.

2. Analyseur selon la revendication 1 dans lequel la caractéristique désirée inclut au moins l'un des suivants : le ferromagnétisme et le paramagnétisme.

3. Analyseur selon la revendication 1 ou 2 dans lequel l'échantillon, sur son passage sur la voie, amène une première impulsion à être générée lorsque l'échantillon entre dans le champ électromagnétique et une seconde impulsion à être générée lorsque l'échantillon quitte le champ électromagnétique, et dans lequel le processeur est configuré pour combiner les première et seconde impulsions de manière électronique pour générer ledit différentiel.

4. Analyseur selon l'une quelconque des revendications 1 à 3 dans lequel les bobines sont orientées verticalement de sorte qu'un échantillon tombant sous l'action de la gravité se déplace dans une direction axiale successivement à travers les bobines alignées.

5. Analyseur selon l'une quelconque des revendications 1 à 4 dans lequel les première et seconde bobines annulaires de détection (212,214) entourent au moins une partie de la voie (90) et sont espacées dans un sens axial l'une de l'autre de sorte qu'un échantillon se déplaçant le long de la voie se déplace d'abord à travers la première bobine de détection (212), ensuite à travers la bobine d'excitation (216) et ensuite à travers la seconde bobine de détection (214).

6. Analyseur selon l'une quelconque des revendications 1 à 5 dans lequel la bobine d'excitation (16) est située entre la première bobine de détection (12) et la seconde bobine de détection (14) dans une configuration radiale.

7. Système comprenant un analyseur selon l'une quelconque des revendications 1 à 6 qui inclut en outre un système de manutention de matériaux (60) qui est en partie positionné en amont des bobines, pour alimenter des échantillons le long de la voie (90) à travers les bobines.

8. Système selon la revendication 7 dans lequel le système de manutention de matériaux (60) inclut :
a) un appareil (62) de séparation d'un flux de particules rocheuses produites par une machine d'extraction (58) en matériau fin, qui est dirigé vers des stériles, et en débris rocheux qui sont plus grossiers que le matériau fin ;
b) une première structure de guidage (64), réalisée à partir d'un matériau non magnétique, qui présente une extrémité supérieure reliée à l'appareil et une extrémité inférieure et qui entoure des débris rocheux tombant, depuis l'appareil, sous l'action de la gravité ;
c) un contrôleur (72) à l'extrémité inférieure qui collecte les débris rocheux tombant et qui amène alors les débris rocheux à se déplacer à une vitesse commandée le long de la voie (90) par laquelle les débris sont présentés aux bobines après quoi les débris rocheux quittent les bobines au niveau d'une sortie ; et
d) une seconde structure de guidage (68), réalisée à partir d'un matériau non magnétique, qui présente une extrémité supérieure (92) en relation avec la sortie de la voie (90) et une extrémité inférieure (94) et qui entoure des débris rocheux tombant, depuis la voie, sous l'action de la gravité.

9. Procédé d'analyse d'un échantillon de débris rocheux pour détecter une caractéristique dans l'échantillon, le procédé incluant les étapes consistant à :
définir une voie le long de laquelle l'échantillon est déplacé,
établir un champ électromagnétique dans au moins une partie de la voie,
détecter une première variation dans le champ électromagnétique, à un premier emplacement, causée par le passage de l'échantillon le long de la voie,
générer un premier signal qui est représentatif de la première variation,
détecter une seconde variation dans le champ électromagnétique, à un second emplacement qui est espacé du premier emplacement, causée par le passage de l'échantillon le long de la voie,
générer un second signal qui est représentatif de la seconde variation,
**caractérisé en ce qu'**il comprend en outre
produire un signal de sortie qui est dépendant d'un différentiel entre les premier et second signaux, dans lequel des signaux d'interférence de signal de sortie sont sensiblement éliminés, et lequel signal de sortie est représentatif de la susceptibilité magnétique de l'échantillon.

10. Procédé selon la revendication 9 dans lequel les premier et second emplacements sont positionnés sur la voie et sont espacés l'un de l'autre.

11. Procédé selon la revendication 9 dans lequel les premier et second emplacements sont situés dans un plan qui est transverse à la voie.

12. Procédé selon l'une quelconque des revendications 9 à 11 dans lequel l'échantillon, sur son passage le long de la voie, génère une première impulsion lorsque l'échantillon entre dans le champ électromagnétique et une seconde impulsion lorsque l'échantillon quitte le champ électromagnétique et qui inclut l'étape consistant à combiner les première et seconde impulsions de manière électronique pour générer ledit différentiel.

13. Procédé selon l'une quelconque des revendications 9 à 12 dans lequel l'échantillon est autorisé à tomber sous l'action de la gravité le long de la voie.

14. Procédé selon l'une quelconque des revendications 9 à 13 dans lequel l'échantillon est l'un d'une pluralité d'échantillons qui sont dirigés successivement, dans un flux continu, le long de la voie.

15. Procédé selon la revendication 14, qui inclut les étapes consistant à utiliser une machine d'extraction pour produire la pluralité d'échantillons, enlever, au moins, de la poussière de la pluralité d'échantillons, et amener les échantillons à se déplacer, successivement, à travers le champ électromagnétique le long d'une voie, dans lequel une pluralité de signaux de sortie respectifs sont produits.

16. Procédé selon la revendication 15 qui inclut les étapes consistant à dériver des données de mesure à partir des signaux de sortie respectifs, comparer les données de mesure à des données de référence pour fournir au moins un signal de commande, et utiliser l'au moins un signal de commande pour commander le fonctionnement d'une machine d'extraction.

17. Procédé selon la revendication 16, dans lequel la machine d'extraction est la machine d'extraction utilisée pour produire la pluralité d'échantillons de débris rocheux.

18. Produit programme d'ordinateur chargeable directement dans la mémoire interne d'un ordinateur numérique, comprenant des portions de code de logiciel configurées pour amener un analyseur selon les revendications 1-8 à effectuer les étapes consistant à :
établir un champ électromagnétique dans au moins une partie de la voie,
détecter une première variation dans le champ électromagnétique, à un premier emplacement, causée par le passage de l'échantillon le long de la voie,
générer un premier signal qui est représentatif de la première variation,
détecter une seconde variation dans le champ électromagnétique, à un second emplacement qui est espacé du premier emplacement, causée par le passage de l'échantillon le long de la voie,
générer un second signal qui est représentatif de la seconde variation, et
produire un signal de sortie qui est dépendant d'un différentiel entre les premier et second signaux, dans lequel des signaux d'interférence de signal de sortie sont sensiblement éliminés, et lequel signal de sortie est représentatif de la susceptibilité magnétique de l'échantillon, lorsque ledit produit est exécuté sur un ordinateur.
